# DEMANDE DE BREVET EUROPEEN

(11) **EP 1 040 818 A1**
(43) Date de publication de la demande: **04.10.2000**
(21) Numéro de dépôt: 00400545.0
(22) Date de dépôt: 29.02.2000
(51) Int. Cl.: A61K 7/13

(54) **Composition de teinture d'oxydation des fibres kératiniques et procédé de teinture mettant en oeuvre cette composition**

(30) Priorité: 29.03.1999 FR 9903890
(71) Demandeur: L'OREAL, 75008 Paris (FR)
(72) Inventeur: Audousset, Marie-Pascale, 92600 Asnieres (FR)
(74) Mandataire: Goulard, Sophie

(57) **Abrégé**

L'invention a pour objet une composition pour la teinture d'oxydation des fibres kératiniques, et en particulier des fibres kératiniques humaines telles que les cheveux comprenant, dans un milieu approprié pour la teinture, au moins une première base d'oxydation choisie parmi la paraphénylènediamine, la paratoluylènediamine et leurs sels d'addition avec un acide, au moins une deuxième base d'oxydation choisie parmi la N,N-bis-(β-hydroxyéthyl) paraphénylènediamine, les pyrazolo-[1,5-a]-pyrimidines, les para-aminophénols et leurs sels d'addition avec un acide, et au moins un coupleur choisi parmi les pyrazolo-[3,2-c]-1,2,4-triazoles, ainsi que le procédé de teinture mettant en oeuvre cette composition.

## Description

L'invention a pour objet une composition pour la teinture d'oxydation des fibres kératiniques, et en particulier des fibres kératiniques humaines telles que les cheveux comprenant, dans un milieu approprié pour la teinture, au moins une première base d'oxydation choisie parmi la paraphénylènediamine, la paratoluylènediamine et leurs sels d'addition avec un acide, au moins une deuxième base d'oxydation choisie parmi la N,N-bis-(β-hydroxyéthyl) paraphénylènediamine, les pyrazolo-[1,5-a]-pyrimidines, les para-aminophénols et leurs sels d'addition avec un acide, et au moins un coupleur choisi parmi les pyrazolo-[3,2-c]-1,2,4-triazoles, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demande de brevet FR-A-2 750 048, des compositions pour la teinture d'oxydation des fibres kératiniques contenant, à titre de base d'oxydation, des pyrazolo-[1,5-a]-pyrimidines, éventuellement associées avec un ou plusieurs coupleurs. Il a également déjà été proposé, notamment dans la demande de brevet FR-A-2 156 527, des compositions pour la teinture d'oxydation des fibres kératiniques contenant, à titre de base d'oxydation certains dérivés de la paraphénylènediamine comme par exemple la N,N-bis-(β-hydroxyéthyl) paraphénylènediamine, éventuellement associées avec un ou plusieurs coupleurs. Cependant, les colorations obtenues en mettant en oeuvre ces compositions tinctoriales ne sont pas toujours assez puissantes, chromatiques ou résistantes aux différentes agressions que peuvent subir les cheveux.

Or, la Demanderesse vient maintenant de découvrir qu'il est possible d'obtenir de nouvelles teintures, capables de conduire à des colorations aux nuances variées, chromatiques, puissantes, esthétiques, peu sélectives et résistant bien aux diverses agressions que peuvent subir les fibres, en associant au moins une première base d'oxydation choisie parmi la paraphénylènediamine, la paratoluylènediamine, et leurs sels d'addition avec un acide, au moins une deuxième base d'oxydation choisie parmi la N,N-bis-(β-hydroxyéthyl) paraphénylènediamine, les pyrazolo-[1,5-a]-pyrimidines, les para-aminophénols, et leurs sels d'addition avec un acide, et au moins un coupleur choisi parmi les pyrazolo-[3,2-c]-1,2,4-triazoles.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :
- au moins une première base d'oxydation choisie parmi la paraphénylènediamine, la paratoluylènediamine, et leurs sels d'addition avec un acide,
- au moins une deuxième base d'oxydation choisie parmi la N,N-bis-(β-hydroxyéthyl) paraphénylènediamine, les para-aminophénols, les pyrazolo-[1,5-a]-pyrimidines, et leurs sels d'addition avec un acide ou avec une base,
- au moins un coupleur choisi parmi les pyrazolo-[3,2-c]-1,2,4-triazoles de formule (I) suivante, et leurs sels d'addition avec un acide : 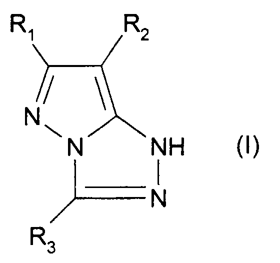 dans laquelle :
   - R₁ et R₃, identiques ou différents, représentent un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle (tel que phényle ou naphtyle), éventuellement substitué par 1 ou 2 radicaux R tels que précédemment définis ; un hétérocycle à 5 ou 6 chaînons possédant au moins un atome d'azote, d'oxygène ou de soufre (tel que pyridyle, quinolyle, pyrrolyle, morpholyle, furanyle, tétrahydrofuranyle, pyrazolyle, triazolyle, tétrazolyle, thiazolyle, oxazolyle, imidazolyle ou thiadiazolyle), éventuellement substitué par 1 ou 2 radicaux R tels que définis précédemment ;
      lorsque R₁ et/ou R₃ désignent un radical alkyle, un radical aryle ou un hétérocycle à 5 ou 6 chaînons tel que défini ci-dessus, il peut être relié à l'atome de carbone du noyau par l'intermédiaire d'un atome d'oxygène, d'azote ou de soufre (dans ce cas, R₁ devient XR₁ avec X = O, NH, S) ;
      R₁ et/ou R₃ peuvent en outre désigner un atome d'halogène (tel que brome, chlore ou fluor) ; un radical acyle ; un radical sulfonyle ; un radical sulfinyle ; un radical phosphonyle, un radical carbamoyle ; un radical sulfamoyle ; un radical cyano ; un radical siloxy, un radical amino ; un radical acylamino ; un radical acyloxy; un radical carbamoyloxy ; un radical sulfonamide ; un radical imide ; un radical uréido ; un radical sulfamoylamino ; un radical alcoxycarbonylamino ; un radical aryloxycarbonylamino ; un radical alcoxycarbonyle; un radical aryloxycarbonyle; un radical carboxyle ;
   - R₂ représente un atome d'hydrogène ; un atome d'halogène tel que brome, chlore ou fluor ; un groupe acétylamido ; un radical alcoxy (tel que par exemple : méthoxy, éthoxy, propyloxy, benzyloxy, méthoxyéthoxy, phénoxyéthoxy, 2-cyanoéthoxy, phénéthyloxy, p-chlorobenzyloxy, méthoxyéthylcarbamoylméthoxy) ; un radical aryloxy (tel que par exemple : phénoxy, 4-méthoxyphénoxy, 4-nitrophénoxy, 4-cyanophénoxy, 4-méthanesulfona-midophénoxy, 4-méthanesulfonylphénoxy, 3-méthylphénoxy, 1-naphtyloxy) ; un radical acyloxy (tel que par exemple : acétoxy, propanoyloxy, benzoyloxy, 2,4-dichlorobenzoyloxy, éthoxyalkyloxy, pyruviloyloxy, cinnamoyloxy, myristoyloxy) ; un radical arylthio (tel que par exemple : phénylthio, 4-carboxy-phénylthio, 2-éthoxy 5-tert-butylphénylthio, 2-carboxyphénylthio, 4-méthane-sulfonyl-phénylthio) ; un radical alkylthio (tel que par exemple : méthylthio, éthylthio, propylthio, butylthio, 2-cyanoéthylthio, benzylthio, phénéthylthio, 2-(diéthylamino) éthylthio, éthoxyéthylthio, phénoxyéthylthio) ; un radical hétéroarylthio (tel que par exemple : 5-phényl 2,3,4,5-tétrazolylthio, 2-benzothiazolylthio) ; un radical hétéroaryloxy (tel que par exemple : 5-phényl 2,3,4,5-tétrazolyloxy, 2-benzo-thiazolyloxy) ; un radical thiocyano ; un radical N,N-diéthyl thiocarbonylthio ; un radical dodécyl-oxythio carbonylthio ; un radical benzènesulfonamido ; un radical N-éthyltoluène sulfonamido ; un radical pentafluorobutanamido ; un radical 2,3,4,5,6-pentafluorobenzamido ; un radical p-cyanophényluréido, un radical N,N-diéthylsulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido ; un radical NR^{III}R^{IV} avec R^{III} et R^{IV} représentant, identiques ou différents, un alkyle en C₁-C₄ ; un hydroxyalkyle ; un carboxyle ; ou un radical alcoxycarboxylique.

La composition tinctoriale conforme à l'invention conduit à des colorations dans des nuances variées, chromatiques, puissantes, esthétiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux.

L'invention a également pour objet un procédé de teinture d'oxydation des fibres kératiniques mettant en oeuvre cette composition tinctoriale.

Parmi les composés de formule (I) ci-dessus, on peut tout particulièrement citer :
- le 3-méthyl pyrazolo [3,2-c]-1,2,4-triazole,
- le 3-méthylsulfinyl-6-phényl-pyrazolo [3,2-c]-1,2,4-triazole,
- le 3-éthyl pyrazolo [3,2-c]-1,2,4-triazole,
- le 3-isopropyl pyrazolo [3,2-c]-1,2,4-triazole,
- le 3-phényl pyrazolo [3,2-c]-1,2,4-triazole,
- le 3-(2'-aminoéthyl) pyrazolo [3,2-c]-1,2,4-triazole,
- le 3-(2'-hydroxyéthyl) pyrazolo [3,2-c]-1,2,4-triazole,
- le 6-méthyl-3-éthyl pyrazolo [3,2-c]-1,2,4-triazole,
- le 3,6-diméthyl- pyrazolo [3,2-c]-1,2,4-triazole,
- le 6-méthyl-3-isopropyl- pyrazolo [3,2-c]-1,2,4-triazole,
- le 6-méthyl-3-phényl- pyrazolo [3,2-c]-1,2,4-triazole,
- le 6-méthyl-3-(2'-aminoéthyl)- pyrazolo [3,2-c]-1,2,4-triazole,
- le 6-méthyl-3-(2'hydroxyéthyl)- pyrazolo [3,2-c]-1,2,4-triazole,
- le 6-méthyl-3-méthylthio- pyrazolo [3,2-c]-1,2,4-triazole,
- le 6-phényl-3-méthyl- pyrazolo [3,2-c]-1,2,4-triazole,
- le 6-phényl-3-éthyl- pyrazolo [3,2-c]-1,2,4-triazole,
- le 6-isopropyl-3-éthyl pyrazolo [3,2-c]-1,2,4-triazole,
- le 6-phényl-3-isopropyl- pyrazolo [3,2-c]-1,2,4-triazole,
- le 6-phényl-3-phényl- pyrazolo [3,2-c]-1,2,4-triazole,
- le 6-phényl-3-(2'-aminoéthyl)-pyrazolo [3,2-c]-1,2,4-triazole,
- le 6-phényl-3-(2'-hydroxyéthyl)- pyrazolo [3,2-c]-1,2,4-triazole,
- le 6-phényl-3-méthylthio- pyrazolo [3,2-c]-1,2,4-triazole,
- le 6-éthylthio-3-méthyl- pyrazolo [3,2-c]-1,2,4-triazole,
- le 6-éthylthio-3-éthyl- pyrazolo [3,2-c]-1,2,4-triazole,
- le 6-éthylthio-3-isopropyl- pyrazolo [3,2-c]-1,2,4-triazole,
- le 6-éthylthio-3-phényl- pyrazolo [3,2-c]-1,2,4-triazole,
- le 6-éthylthio-3-(2'-aminoéthyl)- pyrazolo [3,2-c]-1,2,4-triazole,
- le 6-éthylthio-3-(2'-hydroxyéthyl)- pyrazolo [3,2-c]-1,2,4-triazole,
- le 6-trifluorométhyl-3-méthylthio- pyrazolo [3,2-c]-1,2,4-triazole,
- le 6-trifluorométhyl- pyrazolo [3,2-c]-1,2,4-triazole,
- le 6-carboxy-3-méthyl- pyrazolo [3,2-c]-1,2,4-triazole,
- le 6-carboxy-3-éthyl- pyrazolo [3,2-c]-1,2,4-triazole,
- le 6-carboxy-3-isopropyl- pyrazolo [3,2-c]-1,2,4-triazole,
- le 6-carboxy-3-phényl- pyrazolo [3,2-c]-1,2,4-triazole,
- le 6-carboxy-3-(2'amino-éthyl)- pyrazolo [3,2-c]-1,2,4-triazole,
- le 6-carboxy-3-(2'-hydroxyéthyl)- pyrazolo [3,2-c]-1,2,4-triazole,
- le 7-chloro-3,6-diméthylpyrazolo[3,2-c]-1,2,4-triazole,
- le 7-méthoxycarbonyl-3,6-diméthylpyrazolo [3,2-c]-1,2,4-triazole,
et leurs sels d'addition avec un acide.

Les composés de formule (I) utilisés à titre de coupleur dans la composition tinctoriale conforme à l'invention, leurs intermédiaires de synthèse et leurs procédés de préparation sont décrits dans les demandes de brevets et brevets suivants : FR 2 075 583, EP-A-119 860, EP-A-285 274, EP-A-244 160, EP-A-578 248, GB 1 458 377, US 3 227 554, US 3 419 391, US 3 061 432, US 4 500 630, US 3 725 067, US 3 926 631, US 5 457 210, JP 84/99437, JP 83/42045, JP 84/162548, JP 84/171956, JP 85/33552, JP 85/43659, JP 85/172982, JP 85/190779, ainsi que dans les publications suivantes : Chem. Ber. **32**, 797 (1899), Chem. Ber. **89**, 2550, (1956), J. Chem. Soc. Perkin trans I, 2047, (1977), J. Prakt. Chem., 320, 533, (1978), J. fur Chem., 326(5), 829, (1984).

Parmi les para-aminophénols utilisables à titre de deuxième base d'oxydation dans la composition tinctoriale conforme à l'invention, on peut notamment citer les composés répondant à la formule (II) suivante, et leurs sels d'addition avec un acide : 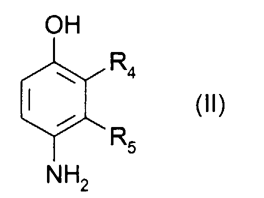 dans laquelle :
- R₄ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₅ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₄ ou R₅ représente un atome d'hydrogène.

Parmi les para-aminophénols de formule (II) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les pyrazolo-[1,5-a]-pyrimidines utilisables à titre de deuxième base d'oxydation dans la composition tinctoriale conforme à l'invention, on peut notamment citer les composés de formule (III) suivante, et leurs sels d'addition avec un acide ou avec une base : 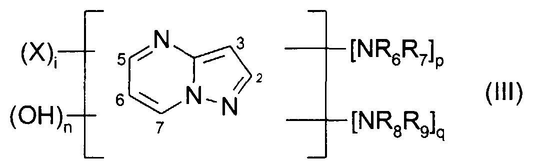 dans laquelle :
- R₆ R₇, R₈ et R₉ désignent , identiques ou différents, un atome d'hydrogène ; un radical alkyle en C₁-C₄ ; un radical aryle ; un radical hydroxyalkyle en C₁-C₄ ; un radical polyhydroxyalkyle en C₂-C₄ ; un radical (C₁-C₄)alcoxy alkyle en C₁-C₄ ; un radical amino alkyle en C₁-C₄ ; un radical amino alkyle en C₁-C₄ dont l'amine est protégée par un radical acétyle, uréido, ou sulfonyl ; un radical (C₁-C₄)alkyl amino alkyle en C₁-C₄ ; un radical di-[(C₁-C₄)alkyl] amino alkyle en C₁-C₄ dans lequel les dialkyles peuvent former un cycle aliphatique ou hétérocyclique à 5 ou 6 chaînons ; un radical hydroxy(C₁-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ dans lequel les dialkyles peuvent former un cycle aliphatique ou hétérocyclique à 5 ou 6 chaînons, un radical hydroxy(C₁-C₄)alkyl- ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, ou un groupe acide sulfonique ;

- i vaut 0, 1, 2 ou 3 ;
- p vaut 0 ou 1 ;
- q vaut 0 ou 1 ;
- n vaut 0 ou 1 ;
sous réserve que:
- (i) la somme p + q est différente de 0 ;
- (ii) lorsque p + q est égal à 2, alors n vaut 0 et les groupes NR₆R₇ et NR₈R₉ occupent les positions (2,3) ; (5,6) ; (6,7) ; (3,5) ou (3,7) ;
- (iii) lorsque p + q est égal à 1 alors n vaut 1 et le groupe NR₆R₇ (ou NR₈R₉) et le groupe OH occupent les positions (2,3) ; (5,6) ; (6,7) ; (3,5) ou (3,7).

Les pyrazolo-[1,5-a]-pyrimidines de formule (III) utilisables à titre de deuxième base d'oxydation dans la composition tinctoriale conforme à l'invention sont des composés connus et décrits dans la demande de brevet FR-A-2 750 048 dont le contenu fait partie intégrante de la présente demande.

Parmi les pyrazolo-[1,5-a]-pyrimidines de formule (III) ci-dessus, on peut notamment citer :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine ;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl)-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
- la 3-amino-5-méthyl-7-imidazolylpropylamino pyrazolo-[1,5-a]-pyrimidine ;
et leurs sels d'addition avec un acide ou avec une base.

La paraphénylènediamine et/ou la paratoluylènediamine, et/ou le ou leurs sels d'addition avec un acide représentent de préférence de 0,0005 à 12 % en poids environ du poids total de la composition tinctoriale conforme à l'invention, et encore plus préférentiellement de 0,005 à 6 % en poids environ de ce poids.

La N,N-bis-(β-hydroxyéthyl) paraphénylènediamine et/ou le ou les para-aminophénols et/ou la ou les pyrazolo-[1,5-a]-pyrimidines, et/ou le ou leurs sels d'addition avec un acide ou avec une base représentent de préférence de 0,0005 à 12 % en poids environ du poids total de la composition tinctoriale conforme à l'invention, et encore plus préférentiellement de 0,005 à 6 % en poids environ de ce poids.

Le ou les composés de formule (I) et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention, peut en outre, en plus du ou des composés de formule (I), contenir un ou plusieurs coupleurs additionnels choisis de préférence parmi les méta-aminophénols substitués, les méta-diphénols tels que la résorcine et ses dérivés, les méta-phénylènediamines, les coupleurs hétérocycliques, et leurs sels d'addition avec un acide.

Parmi les méta-aminophénols substitués utilisables à titre de coupleur additionnel dans la composition tinctoriale conforme à l'invention, on peut plus particulièrement citer le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, 3-amino 6-chloro phénol, le 3-amino 6-bromo phénol, le 3-(β-aminoéthyl)amino 6-chloro phénol, le 3-(p-hydroxyéthyl)amino 6-chloro phénol et leurs sels d'addition avec un acide.

Parmi les coupleurs hétérocycliques pouvant être utilisés à titre de coupleurs additionnels dans la composition tinctoriale conforme à l'invention, on peut notamment citer les dérivés d'indole, les dérivés de pyridine, les dérivés de benzimidazole, les dérivés de benzomorpholine, les dérivés de sésamol, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

Parmi les dérivés d'indoles, on peut en particulier citer le 4-hydroxy indole, le 6-hydroxy indole, le 7-amino indole, le 6-amino indole, le 7-hydroxy indole, le 7-éthyl 6-(β-hydroxyéthyl)amino indole, le 4-amino indole, le 6-hydroxy 1-méthyl indole, le 5,6-dihydroxy indole, le 4-hydroxy 1-N-méthyl indole, le 4-hydroxy 2-méthyl indole, le 4-hydroxy 5-méthyl indole, le 1-N-(β-hydroxyéthyl) 4-hydroxy indole, le 4-hydroxy 1-N-(β-hydroxypropyl) indole, le 1-N-(β,γ-dihydroxypropyl) 4-hydroxy indole, le 4-hydroxy 1-N-(β-hydroxyéthyl) 5-méthyl indole, le 1-N-(γ-diméthylaminopropyl) 4-hydroxy indole, et leur sels d'addition avec un acide.

Parmi les dérivés de pyridine, on peut en particulier citer la 2-amino 3-hydroxy pyridine, et ses sels d'addition avec un acide.

Parmi les dérivés de benzimidazole on peut plus particulièrement citer le 4-hydroxy benzimidazole, le 4-amino benzimidazole, le 4-hydroxy 7-méthyl benzimidazole, le 4-hydroxy 2-méthyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy 1-méthyl benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sels d'addition avec un acide.

Parmi les dérivés de benzomorpholine on peut plus particulièrement citer la 6-hydroxy 1,4-benzomorpholine, la N-méthyl 6-hydroxy 1,4-benzomorpholine, la 6-amino 1,4-benzomorpholine, et leurs sels d'addition avec un acide.

Parmi les dérivés de sésamol on peut particulièrement citer le 2-bromo 4,5-méthylènedioxy phénol, la 2-méthoxy 4,5-méthylènedioxy aniline, le 2-(β-hydroxyéthyl)amino 4,5-méthylènedioxy benzène, et leurs sels d'addition avec un acide.

Parmi les dérivés pyrrolo-azoliques on peut plus particulièrement citer les composés décrits dans les demandes de brevets et brevets suivants : US 5 256 526, EP-A-557 851, EP-A-578 248, EP-A-518 238, EP-A-456 226, EP-A-488 909, EP-A-488 248, et dans les publications suivantes :
- D.R. Liljegren Ber. 1964, 3436 ;
- E.J. Browne, J.C.S., 1962, 5149 ;
- P. Magnus, J.A.C.S., 1990, 112, 2465 ;
- P. Magnus, J.A.C.S., 1987, 109, 2711 ;
- Angew. Chem. 1960, 72, 956 ;
- et Rec. Trav. Chim. 1961, 80, 1075 ; dont les enseignements font partie intégrante de la présente demande.

A titre de dérivés pyrrolo-azoliques, on peut tout particulièrement citer :
- le 5-cyano-4-éthoxycarbonyl-8-méthyl pyrrolo [1,2-b]-1,2,4-triazole,
- le 5-cyano-8-méthyl-4-phényl pyrrolo [1,2-b]-1,2,4-triazole,
- le 7-amido-6-éthoxycarbonyl pyrrolo [1,2-a]- benzimidazole,
et leurs sels d'addition avec un acide.

Parmi les dérivés imidazolo-azoliques on peut plus particulièrement citer les composés décrits dans les demandes de brevets et brevets suivants : US 5,441,863 ; JP 62-279 337 ; JP 06-236 011 et JP 07-092 632, dont les enseignements font partie intégrante de la présente demande.

A titre de dérivés imidazolo-azoliques, on peut tout particulièrement citer:
- le 7,8-dicyano-imidazolo- [3,2-a]- imidazole,
- le 7,8-dicyano-4-méthyl-imidazolo- [3,2-a]- imidazole,
et leurs sels d'addition avec un acide.

Parmi les dérivés pyrazolo-pyrimidiniques on peut plus particulièrement citer les composés décrits dans la demande de brevet suivante : EP-A-304001 dont l'enseignement fait partie intégrante de la présente demande.

A titre de dérivés pyrazolo-pyrimidiniques, on peut tout particulièrement citer :
- le pyrazolo [1,5-a] pyrimidin-7-one,
- le 2,5-diméthyl pyrazolo [1,5-a] pyrimidin-7-one,
- le 2-méthyl-6-éthoxycarbonyl pyrazolo [1,5-a] pyrimidin-7-one,
- le 2-méthyl-5-méthoxyméthyl pyrazolo [1,5-a] pyrimidin-7-one,
- le 2-ter-butyl-5-trifluorométhyl pyrazolo [1,5-a] pyrimidin-7-one,
- 2,7-diméthyl pyrazolo [1,5-a] pyrimidin-5-one,
et leurs sels d'addition avec un acide.

Parmi les dérivés de pyrazolin-3,5-diones on peut plus particulièrement citer les composés décrits dans les demandes de brevets et brevets suivants : JP 07-036159, JP 07-084348 et US 4 128 425, et dans les publications suivantes :
- L. WYZGOWSKA, Acta. Pol. Pharm. 1982, 39 (1-3), 83.
- E. HANNIG, Pharmazie, 1980, 35 (4), 231
- M. H. ELNAGDI, Bull. Chem. Soc. Jap., 46 (6), 1830, 1973
- G. CARDILLO, Gazz. Chim. Ital. 1966, 96, (8-9), 973.
dont les enseignements font partie intégrante de la présente demande.

A titre de dérivés de pyrazolin-3,5-diones, on peut tout particulièrement citer :
- la 1,2-diphényl pyrazolin-3,5-dione,
- la 1,2-diéthyl pyrazolin-3,5-dione,
et leurs sels d'addition avec un acide.

Parmi les dérivés pyrrolo-[3,2-d]-oxazoliques on peut plus particulièrement citer les composés décrits dans la demande de brevet JP 07 325 375 dont l'enseignement fait partie intégrante de la présente demande.

Parmi les dérivés pyrazolo-[3,4-d]-thiazoliques on peut plus particulièrement citer les composés décrits dans la demandes de brevet JP 07 244 361 et dans J. Heterocycl. Chem. 16, 13, (1979).

Parmi les dérivés S-oxyde-thiazolo-azoliques et S,S-dioxyde-thiazolo-azoliques on peut plus particulièrement citer les composés décrits dans les documents suivants :
- JP 07 09 84 89 ;
- Khim. Geterotsilk. Soedin, 1967, p. 93 ;
- J. Prakt. Chem., 318, 1976, p. 12 ;
- Indian J. Heterocycl. Chem. 1995, 5 (2), p. 135 ;
- Acta. Pol. Pharm. 1995, 52 (5), 415 ;
- Heterocycl. Commun. 1995, 1 (4), 297 ;
- Arch. Pharm. (Weinheim, Ger.), 1994, 327 (12), 825.

Lorsqu'ils sont présents, le ou les coupleurs additionnels représentent de préférence de 0,0001 à 12 % en poids environ du poids total de la composition tinctoriale et encore plus préférentiellement de 0,005 à 6 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates. Les sels d'addition avec une base utilisables dans le cadre des compositions tinctoriales de l'invention sont notamment ceux obtenus avec la soude, la potasse, l'ammoniaque ou les amines.

Le milieu approprié pour la teinture (ou support) de la composition tinctoriale conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (IV) suivante : 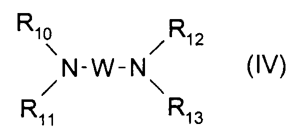 dans laquelle W est un reste propylène éventuellement substitué par un groupement hydroxyle ou un radical alkyle en C₁-C₄ ; R₁₀, R₁₁, R₁₂ et R₁₃, identiques ou différents, représentent un atome d'hydrogène, un radical alkyle en C₁-C₄ ou hydroxyalkyle en C₁-C₄.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques tels que par exemple des gommes de guar non-ioniques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de poudres, de crèmes, de gels, éventuellement pressurisés, ou sous toute autre forme appropriée pour réaliser une teinture des fibres kératiniques, et notamment des cheveux humains.

L'invention a également pour objet un procédé de teinture des fibres kératiniques, et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, les enzymes telles que les oxydo-réductases à 2 électrons, les peroxydases et les laccases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 A 10 DE TEINTURE

On a préparé les compositions tinctoriales suivantes (teneurs en grammes) :

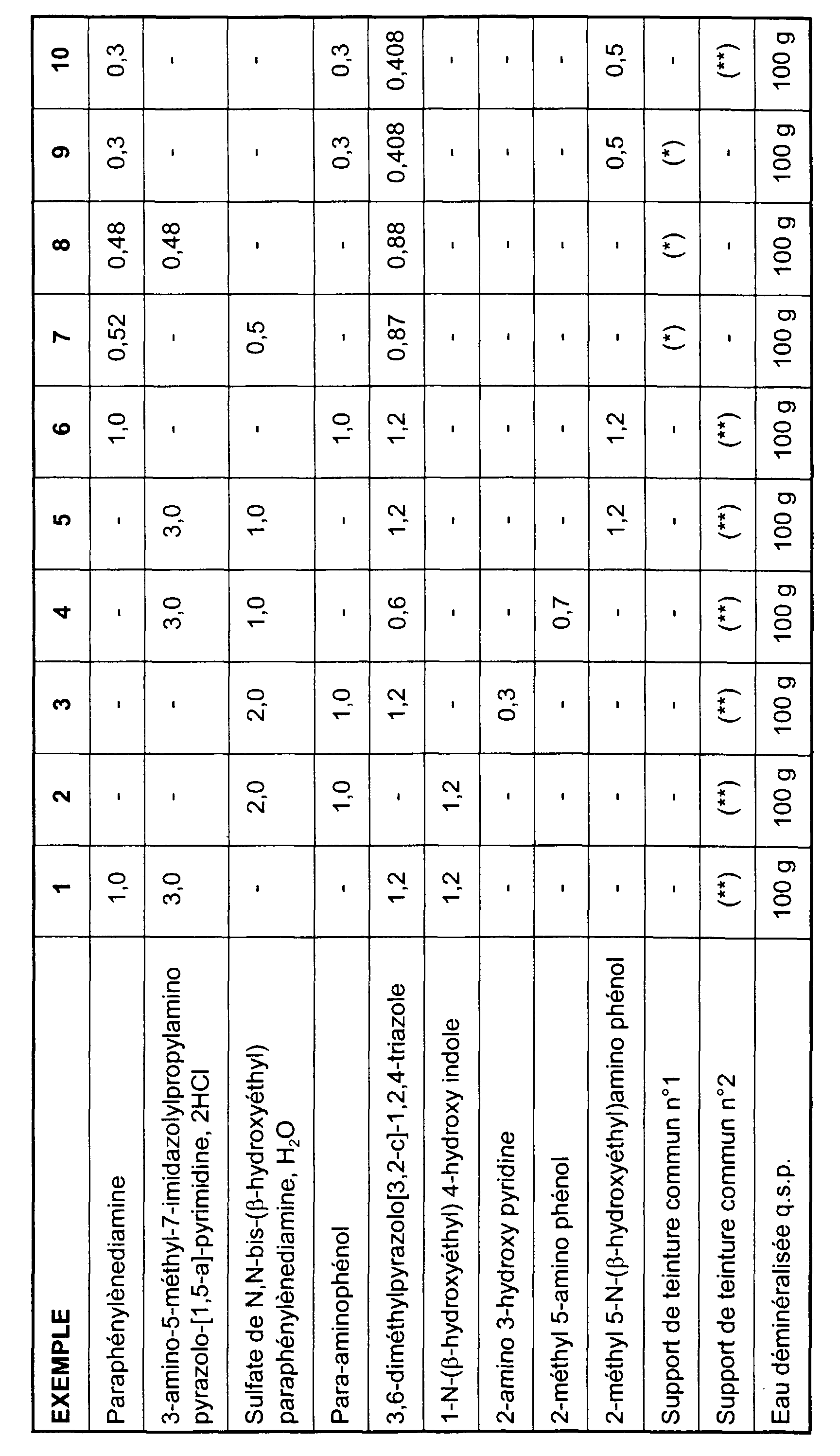

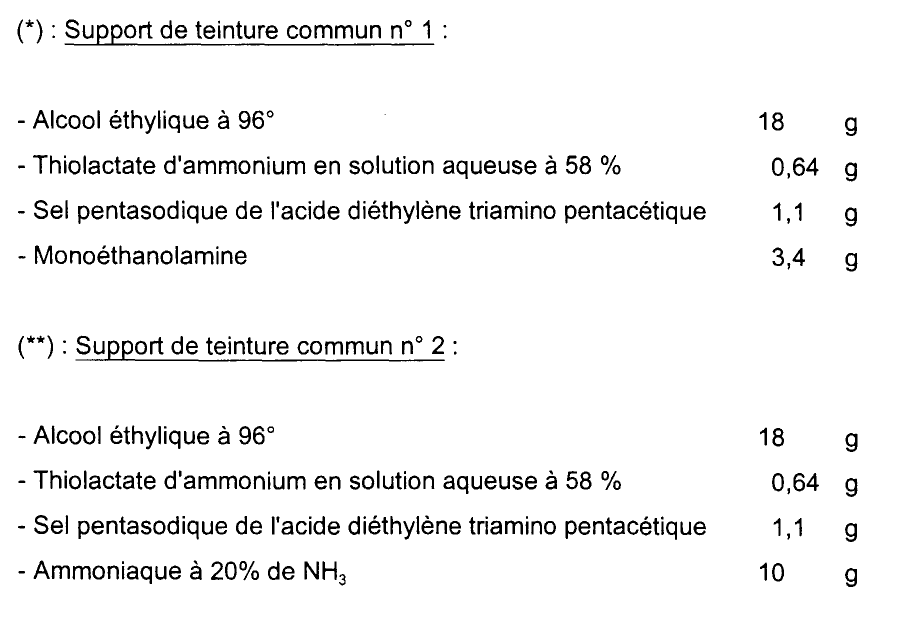

Au moment de l'emploi, on a mélangé poids pour poids chacune des compositions tinctoriales décrites ci-dessus avec une solution de peroxyde d'hydrogène à 20 volumes (6% en poids).

Chacun des mélange ainsi réalisé a été appliqué pendant 30 minutes sur des mèches de cheveux gris naturels à 90 % de blancs. Les mèches ont ensuite été rincées, lavées avec un shampooing standard, rincées à nouveau puis séchées.

Les cheveux ont été teints dans une nuance figurant dans le tableau ci-après :

| **EXEMPLE** | **pH de teinture** | **NUANCE OBTENUE** |
|---|---|---|
| **1** | 10 ± 0,2 | Brun rouge |
| **2** | 10 ± 0,2 | Blond clair violacé |
| **3** | 10 ± 0,2 | Marron clair |
| **4** | 10 ± 0,2 | Blond acajou |
| **5** | 10 ± 0,2 | Blond violacé |
| **6** | 10 ± 0,2 | Blond cuivré |
| **7** | 10 ± 0,2 | Rouge violacé |
| **8** | 10 ± 0,2 | Rouge cuivré |
| **9** | 10 ± 0,2 | Blond foncé cuivré rouge |
| **10** | 10 ± 0,2 | Blond foncé cuivré |

## Revendications

1. Composition pour la teinture d'oxydation des fibres kératiniques et en particulier des fibres kératiniques humaines telles que les cheveux, caractérisée par le fait qu'elle comprend, dans un milieu approprié pour la teinture :
- au moins une première base d'oxydation choisie parmi la paraphénylènediamine, la paratoluylènediamine, et leurs sels d'addition avec un acide,
- au moins une deuxième base d'oxydation choisie parmi la N,N-bis-(β-hydroxyéthyl) paraphénylènediamine, les para-aminophénols, les pyrazolo-[1,5-a]-pyrimidines, et leurs sels d'addition avec un acide ou avec une base,
- au moins un coupleur choisi parmi les pyrazolo-[3,2-c]-1,2,4-triazoles de formule (I) suivante, et leurs sels d'addition avec un acide : 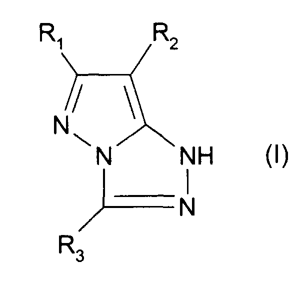 dans laquelle :
- R₁ et R₃, identiques ou différents, représentent un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle, éventuellement substitué par 1 ou 2 radicaux R tels que précédemment définis ; un hétérocycle à 5 ou 6 chaînons possédant au moins un atome d'azote, d'oxygène ou de soufre, éventuellement substitué par 1 ou 2 radicaux R tels que définis précédemment ;
lorsque R₁ et/ou R₃ désignent un radical alkyle, un radical aryle ou un hétérocycle à 5 ou 6 chaînons tel que défini ci-dessus, il peut être relié à l'atome de carbone du noyau par l'intermédiaire d'un atome d'oxygène, d'azote ou de soufre ;
R₁ et/ou R₃ peuvent en outre désigner un atome d'halogène ; un radical acyle ; un radical sulfonyle ; un radical sulfinyle ; un radical phosphonyle, un radical carbamoyle ; un radical sulfamoyle ; un radical cyano ; un radical siloxy, un radical amino ; un radical acylamino ; un radical acyloxy ; un radical carbamoyloxy ; un radical sulfonamide ; un radical imide ; un radical uréido ; un radical sulfamoylamino ; un radical alcoxycarbonylamino ; un radical aryloxycarbonylamino ; un radical alcoxycarbonyle ; un radical aryloxycarbonyle ; un radical carboxyle ;
- R₂ représente un atome d'hydrogène ; un atome d'halogène ; un groupe acétylamido ; un radical alcoxy ; un radical aryloxy ; un radical acyloxy ; un radical arylthio ; un radical alkylthio ; un radical hétéroarylthio ; un radical hétéroaryloxy; un radical thiocyano ; un radical N,N-diéthyl thiocarbonylthio ; un radical dodécyl-oxythio carbonylthio ; un radical benzènesulfonamido ; un radical N-éthyltoluène sulfonamido ; un radical pentafluorobutanamido ; un radical 2,3,4,5,6-pentafluorobenzamido ; un radical p-cyanophényluréido, un radical N,N-diéthyl-sulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido ; un radical NR^{III}R^{IV} avec R^{III} et R^{IV} représentant, identiques ou différents, un alkyle en C₁-C₄ ; un hydroxyalkyle ; un carboxyle ; ou un radical alcoxycarboxylique.

2. Composition selon la revendication 1, caractérisée par le fait que les pyrazolo[3,2-c]-1,2,4-triazoles de formule (I) sont choisis parmi :
- le 3-méthyl pyrazolo [3,2-c]-1,2,4-triazole,
- le 3-méthylsulfinyl-6-phényl-pyrazolo [3,2-c]-1,2,4-triazole,
- le 3-éthyl pyrazolo [3,2-c]-1,2,4-triazole,
- le 3-isopropyl pyrazolo [3,2-c]-1,2,4-triazole,
- le 3-phényl pyrazolo [3,2-c]-1,2,4-triazole,
- le 3-(2'-aminoéthyl) pyrazolo [3,2-c]-1,2,4-triazole,
- le 3-(2'-hydroxyéthyl) pyrazolo [3,2-c]-1,2,4-triazole,
- le 6-méthyl-3-éthyl pyrazolo [3,2-c]-1,2,4-triazole,
- le 3,6-diméthyl- pyrazolo [3,2-c]-1,2,4-triazole,
- le 6-méthyl-3-isopropyl- pyrazolo [3,2-c]-1,2,4-triazole,
- le 6-méthyl-3-phényl- pyrazolo [3,2-c]-1,2,4-triazole,
- le 6-méthyl-3-(2'-aminoéthyl)- pyrazolo [3,2-c]-1,2,4-triazole,
- le 6-méthyl-3-(2'hydroxyéthyl)- pyrazolo [3,2-c]-1,2,4-triazole,
- le 6-méthyl-3-méthylthio- pyrazolo [3,2-c]-1,2,4-triazole,
- le 6-phényl-3-méthyl- pyrazolo [3,2-c]-1,2,4-triazole,
- le 6-phényl-3-éthyl- pyrazolo [3,2-c]-1,2,4-triazole,
- le 6-isopropyl-3-éthyl pyrazolo [3,2-c]-1,2,4-triazole,
- le 6-phényl-3-isopropyl- pyrazolo [3,2-c]-1,2,4-triazole,
- le 6-phényl-3-phényl- pyrazolo [3,2-c]-1,2,4-triazole,
- le 6-phényl-3-(2'-aminoéthyl)-pyrazolo [3,2-c]-1,2,4-triazole,
- le 6-phényl-3-(2'-hydroxyéthyl)- pyrazolo [3,2-c]-1,2,4-triazole,
- le 6-phényl-3-méthylthio- pyrazolo [3,2-c]-1,2,4-triazole,
- le 6-éthylthio-3-méthyl- pyrazolo [3,2-c]-1,2,4-triazole,
- le 6-éthylthio-3-éthyl- pyrazolo [3,2-c]-1,2,4-triazole,
- le 6-éthylthio-3-isopropyl- pyrazolo [3,2-c]-1,2,4-triazole,
- le 6-éthylthio-3-phényl- pyrazolo [3,2-c]-1,2,4-triazole,
- le 6-éthylthio-3-(2'-aminoéthyl)- pyrazolo [3,2-c]-1,2,4-triazole,
- le 6-éthylthio-3-(2'-hydroxyéthyl)- pyrazolo [3,2-c]-1,2,4-triazole,
- le 6-trifluorométhyl-3-méthylthio- pyrazolo [3,2-c]-1,2,4-triazole,
- le 6-trifluorométhyl- pyrazolo [3,2-c]-1,2,4-triazole,
- le 6-carboxy-3-méthyl- pyrazolo [3,2-c]-1,2,4-triazole,
- le 6-carboxy-3-éthyl- pyrazolo [3,2-c]-1,2,4-triazole,
- le 6-carboxy-3-isopropyl- pyrazolo [3,2-c]-1,2,4-triazole,
- le 6-carboxy-3-phényl- pyrazolo [3,2-c]-1,2,4-triazole,
- le 6-carboxy-3-(2'amino-éthyl)- pyrazolo [3,2-c]-1,2,4-triazole,
- le 6-carboxy-3-(2'-hydroxyéthyl)- pyrazolo [3,2-c]-1,2,4-triazole,
- le 7-chloro-3,6-diméthylpyrazolo[3,2-c]-1,2,4-triazole,
- le 7-méthoxycarbonyl-3,6-diméthylpyrazolo [3,2-c]-1,2,4-triazole,
et leurs sels d'addition avec un acide.

3. Composition selon la revendication 1 ou 2, caractérisée par le fait que les para-aminophénols sont choisis parmi les composés de formule (II) suivante, et leurs sels d'addition avec un acide : 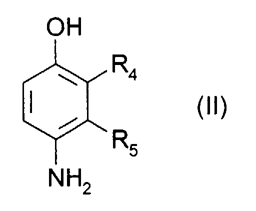 dans laquelle :
- R₄ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₅ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₄ ou R₅ représente un atome d'hydrogène.

4. Composition selon la revendication 3, caractérisée par le fait que les para-aminophénols de formule (II) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

5. Composition selon l'une quelconque des revendications précédentes, caractérisée par le fait que les pyrazolo-[1,5-a]-pyrimidines sont choisies parmi les composés de formule (III) suivante, et leurs sels d'addition avec un acide ou avec une base : 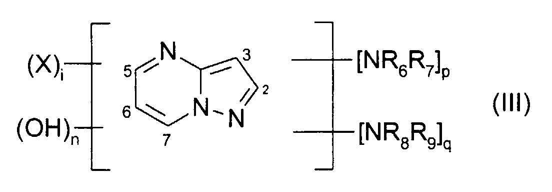 dans laquelle :
- R₆ R₇, R₈ et R₉ désignent , identiques ou différents, un atome d'hydrogène ; un radical alkyle en C₁-C₄ ; un radical aryle ; un radical hydroxyalkyle en C₁-C₄ ; un radical polyhydroxyalkyle en C₂-C₄; un radical (C₁-C₄)alcoxy alkyle en C₁-C₄ ; un radical amino alkyle en C₁-C₄ ; un radical amino alkyle en C₁-C₄ dont l'amine est protégée par un radical acétyle, uréido, ou sulfonyl ; un radical (C₁-C₄)alkyl amino alkyle en C₁-C₄ ; un radical di-[(C₁-C₄)alkyl] amino alkyle en C₁-C₄ dans lequel les dialkyles peuvent former un cycle aliphatique ou hétérocyclique à 5 ou 6 chaînons ; un radical hydroxy(C₁-C₄)alkyl- ; ou un radical di-[hydroxy(C₁-C₄) alkyl]- amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ dans lequel les dialkyles peuvent former un cycle aliphatique ou hétérocyclique à 5 ou 6 chaînons, un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino; un atome d'halogène, un groupe acide carboxylique, ou un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que:
- (i) la somme p + q est différente de 0 ;
- (ii) lorsque p + q est égal à 2, alors n vaut 0 et les groupes NR₆R₇ et NR₈R₉ occupent les positions (2,3) ; (5,6) ; (6,7) ; (3,5) ou (3,7) ;
- (iii) lorsque p + q est égal à 1 alors n vaut 1 et le groupe NR₆R₇(ou NR₈R₉) et le groupe OH occupent les positions (2,3) ; (5,6) ; (6,7) ; (3,5) ou (3,7).

6. Composition selon la revendication 5, caractérisée par le fait que la ou les pyrazolo-[1,5-a]-pyrimidines de formule (III) sont choisies parmi :
- la pyrazolo-[1,5-a]-pyrimidine-3,7-diamine ;
- la 2-méthyl pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol ;
- le 3-amino 5-méthyl pyrazolo-[1,5-a]-pyrimidin-7-ol ;
- le 3-amino pyrazolo-[1,5-a]-pyrimidin-5-ol ;
- le 2-(3-amino pyrazolo-[1,5-a]-pyrimidin-7-ylamino)-éthanol ;
- la 3-amino-7-β-hydroxyéthylamino-5-méthyl-pyrazolo-[1,5-a]-pyrimidine;
- le 2-(7-amino pyrazolo-[1,5-a]-pyrimidin-3-ylamino)-éthanol ;
- le 2-[(3-amino-pyrazolo-[1,5-a]-pyrimidin-7-yl)-(2-hydroxyéthyl)-amino]-éthanol ;
- le 2-[(7-amino-pyrazolo-[1,5-a]-pyrimidin-3-yl)-(2-hydroxyéthyl )-amino]-éthanol ;
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N-7, N-7-tetraméthyl pyrazolo-[1,5-a]-pyrimidine-3,7-diamine ;
- la 3-amino-5-méthyl-7-imidazolylpropylamino pyrazolo-[1,5-a]-pyrimidine ;
et leurs sels d'addition avec un acide ou avec une base.

7. Composition selon l'une quelconque des revendications précédentes, caractérisée par le fait que la paraphénylènediamine et/ou la paratoluylènediamine, et/ou le ou leurs sels d'addition avec un acide représentent de 0,0005 à 12 % en poids du poids total de la composition tinctoriale.

8. Composition selon la revendication 7, caractérisée par le fait que la paraphénylènediamine et/ou la paratoluylènediamine, et/ou le ou leurs sels d'addition avec un acide représentent de 0,005 à 6 % en poids du poids total de la composition tinctoriale.

9. Composition selon l'une quelconque des revendications précédentes, caractérisée par le fait que la N,N-bis-(β-hydroxyéthyl) paraphénylènediamine et/ou le ou les para-aminophénols et/ou la ou les pyrazolo-[1,5-a]-pyrimidines, et/ou le ou leurs sels d'addition avec un acide ou avec une base représentent de 0,0005 à 12 % en poids du poids total de la composition tinctoriale.

10. Composition selon la revendication 9, caractérisée par le fait que la N,N-bis-(β-hydroxyéthyl) paraphénylènediamine et/ou le ou les para-aminophénols et/ou la ou les pyrazolo-[1,5-a]-pyrimidines, et/ou le ou leurs sels d'addition avec un acide ou avec une base représentent de 0,005 à 6 % en poids du poids total de la composition tinctoriale.

11. Composition selon l'une quelconque des revendications précédentes, caractérisée par le fait que le ou les composés de formule (I) et/ou le ou leurs sels d'addition avec un acide représentent de 0,0005 à 12 % en poids du poids total de la composition tinctoriale.

12. Composition selon la revendication 11, caractérisée par le fait que le ou les composés de formule (I) et/ou le ou leurs sels d'addition avec un acide représentent de 0,005 à 6 % en poids du poids total de la composition tinctoriale.

13. Composition selon l'une quelconque des revendications précédentes, caractérisée par le fait qu'elle contient en outre un ou plusieurs coupleurs additionnels choisis parmi les méta-aminophénols substitués, les méta-diphénols, les méta-phénylènediamines, les coupleurs hétérocycliques, et leurs sels d'addition avec un acide.

14. Composition selon la revendication 13, caractérisée par le fait que les méta-aminophénols substitués sont choisis parmi le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, 3-amino 6-chloro phénol, le 3-amino 6-bromo phénol, le 3-(β-aminoéthyl)amino 6-chloro phénol, le 3-(β-hydroxyéthyl)amino 6-chloro phénol et leurs sels d'addition avec un acide.

15. Composition selon la revendication 13, caractérisée par le fait que les coupleurs hétérocycliques sont choisis parmi les dérivés d'indole, les dérivés de pyridine, les dérivés de benzimidazole, les dérivés de benzomorpholine, les dérivés de sésamol, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

16. Composition selon l'une quelconque des revendications précédentes, caractérisée par le fait que le ou les coupleurs additionnels représentent de 0,0001 à 12 % en poids environ du poids total de la composition tinctoriale.

17. Composition selon la revendication 16, caractérisée par le fait que le ou les coupleurs additionnels représentent de 0,005 à 6 % en poids environ du poids total de la composition tinctoriale.

18. Composition selon l'une quelconque des revendications précédentes, caractérisée par le fait que les sels d'addition avec un acide sont choisis parmi les chlorhydrates, les bromhydrates, les sulfates et les tartrates, les lactates et les acétates et que les sels d'addition avec une base sont choisis parmi ceux obtenus avec la soude, la potasse, l'ammoniaque ou les amines.

19. Procédé de teinture des fibres kératiniques et en particulier des fibres kératiniques humaines telles que les cheveux, caractérisé par le fait qu'on applique sur lesdites fibres au moins une composition tinctoriale telle que définie à l'une quelconque des revendications 1 à 18, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

20. Procédé selon la revendication 19, caractérisé par le fait que l'agent oxydant présent dans la composition oxydante est choisi parmi le peroxyde d'hydrogène, le peroxyde d'urée, les bromates de métaux alcalins, les persels, les peracides, et les enzymes.

21. Dispositif à plusieurs compartiments, ou "kit" de teinture à plusieurs compartiments, dont un premier compartiment renferme une composition tinctoriale telle que définie à l'une quelconque des revendications 1 à 18 et un second compartiment renferme une composition oxydante.
